# EUROPEAN PATENT SPECIFICATION

(11) **EP 1 865 323 B1**
(45) Date of publication and mention of the grant of the patent: **01.12.2010**
(21) Application number: 07011195.0
(22) Date of filing: 07.06.2007
(51) Int. Cl.: G01N 33/543

(54) **Sample system for controlling fluid movement between a sample receiving pad and a test strip**
Probensystem zur Kontrolle der Flüssigkeitsbewegung zwischen einem Probenaufnahmetupfer und einem Teststreifen
Système d'échantillons pour le contrôle de mouvements de fluides entre un tampon de réception d'échantillon et une bande d'essai

(30) Priority: 07.06.2006 US 811880 P
(43) Date of publication of application: 12.12.2007
(73) Proprietor: Bayer HealthCare LLC, Tarrytown, NY 10591 (US)
(72) Inventor: Ramel, Urs A., Sunnyvale California 94087 (US); Tay, Dillan, San Jose California 95131 (US)
(74) Representative: Linhart, Angela

(56) References cited:
- WO-A-02/10754
- WO-A-2005/086744
- WO-A-2005/108991
- US-A1- 2002 150 501

## Description

### TECHNICAL FIELD

The present invention relates to fluid flow control systems in test meters.
WO 2005/108991 A2 discloses a structure for controlling fluid flow from a sample receiving pad into a lateral flow assay test system including a lateral flow assay test strip; a sample pad abutting the lateral flow assay test strip; a pinch wall positioned to direct fluid flow from the sample pad to the lateral flow assay test strip; a top support structure positioned on top of the test strip; and a bottom support structure positioned underneath the test strip, wherein each of the top and bottom support structures comprise a plurality of separate spaced-apart support ribs positioned along the length of the test strip. That structure further comprising a pinch wall compressing the sample receiving pad comprising a first portion separating a sample receiving portion of the sample receiving pad from a portion of the sample pad positioned adjacent to the test strip and a second portion separating the sample receiving portion of the sample pad from a portion of the sample pad positioned away from the test strip wherein the first portion of the pinch wall compresses the sample receiving pad to a lesser degree than the second portion of the sample receiving pad.
WO 2005/086744 A2 discloses a combination body fluid analyte meter and cartridge system, having (a) a body fluid analyte meter with a housing; a logic circuit disposed within the housing; a visual display disposed on the housing; and a measurement system disposed within the housing and (b) a cartridge having at least one lateral flow assay test strip, the lateral flow assay strip having (i) a lateral flow transport matrix; (ii) a specific binding assay zone on the transport matrix for receiving a fluid sample and performing a specific binding assay to produce a detectable response and (iii) a general chemical assay zone on the transport matrix for receiving the fluid sample and performing a general chemical assay to produce a detectable response.
US 2002/0150501 A1 discloses a multiple analyte assaying device including a casing having a pocket portion adapted to capture or contain a predetermined volume of fluid specimen to be assayed. Disposed within the casing is a sample receiving pad and one or more reagent test strips. The pocket portion of the casing includes a feed element having multiple feed inlets or alternatively a single feed slot. The feed element provides pressure against the sample receiving pad. The pressure against the pad and a small size of the inlet or inlets are effective in controlling a rate of fluid release of the specimen to the sample receiving pad. The device is structured and adapted to enable a technician to capture the appropriate volume fluid specimen in the pocket portion by submerging or dipping a portion of the device into a fluid-filled collection container for a brief period of time.
WO 02/10754 A2 discloses an assay apparatus comprising a moulded cartridge containing a lateral flow test strip which has a label pad containing an enzyme antibody or antigen conjugate having affinity for an analyte; a capture zone having a capture antibody or antigen having affinity for said analyte; a reagent storage blister; a means for removing sample and/or label pad from the lateral flow test strip; a meter in which the cartridge is inserted enabling the control for reagent release from the reagent storage blister and for the sample and/or label pad removal and a means of providing quantitative measurement of said analyte.

### SUMMARY OF THE INVENTION:

The present invention provides a system for controlling fluid flow movement between a sample receiving pad and a test strip, comprising: a sample receiving pad having first and second portions separated by a narrowed portion; an absorbent pad in contact with the first portion of the sample receiving pad; and a test strip in contact with the second portion of the sample receiving pad.

The present invention is provides novel patentable improvements to the system shown in US 2006/0008847 A1 / WO 2005/108991 A2 (U.S. Patent Application 11/121,972), entitled "Mechanical cartridge with test strip fluid control features for use in a fluid analyte meter", filed May 4, 2005. As such, the present application is related to, but does not claim priority to US 2006/0008847 A1 / WO 2005/108991 A2 (U.S. Patent Application 11/121,972).

In various aspects, the absorbent pad is in overlapping contact with the first portion of the sample receiving pad. For example, the absorbent pad may be positioned on top of (or under) the first portion of the sample receiving pad. An end of the test strip may overlap the second portion of the sample receiving pad.

In various aspects, the sample receiving pad, absorbent pad and test strip are all disposed in a cartridge, and the cartridge has a sample receiving hole positioned over the second portion of the sample receiving pad.

In various aspects, the second portion of the sample receiving pad has an inwardly curved rear edge adjacent to the narrowed portion of the sample receiving pad separating the first and second portions of the sample receiving pad.

In various aspects, the cartridge comprises a pinch wall compressing the sample receiving pad. The pinch wall may optionally comprise: a first portion separating a sample receiving portion of the sample receiving pad from a portion of the sample receiving pad positioned away from the test strip, and a second portion separating the sample receiving portion of the sample receiving pad from a portion of the sample receiving pad positioned adjacent to the test strip, wherein the second portion of the pinch wall compresses the sample receiving pad to a lesser degree than the first portion of the sample receiving pad.

In various aspects, the first and second portions of the pinch wall together continuously surround the sample receiving portion of the sample receiving pad. The pinch wall may preferably compress the sample receiving pad such that fluid received onto the sample receiving pad preferentially tends to flow from the sample receiving portion of the sample receiving pad into the portion of the sample receiving pad positioned adjacent to the test strip, and less so from the sample receiving portion of the sample receiving pad to the portion of the sample receiving pad positioned away from the test strip.

In optional aspects, the first portion of the pinch wall compresses 60 to 90% of the height of the sample receiving pad and the second portion of the pinch wall compresses 2 to 30% of the height of the sample receiving pad. In other optional aspects, the first portion of the pinch wall compresses 70 to 80% of the height of the sample receiving pad and the second portion of the pinch wall compresses 5 to 15% of the height of the sample receiving pad.

In optional aspects, the first portion of the pinch wall comprises a notch adjacent to the absorbent pad to permit fluid flow into the absorbent pad.

### BRIEF DESCRIPTION OF THE DRAWINGS:

Fig. 1 is an exploded perspective view of the system as seen in US 2006/0008847 A1 / WO 2005/108991 A2 (U.S. Patent Application 11/121,972).

Fig. 2 is an exploded perspective view of the present invention, showing novel changes made to the design of Fig. 1.

Fig. 3 is an exploded perspective view of the system of the underside of the cartridge top as seen in US 2006/0008847 A1 / WO 2005/108991 A2 (U.S. Patent Application 11/121,972).

Fig. 4 is an exploded perspective view of the present invention of the underside of the cartridge top, showing novel changes made to the design of Fig. 3.

Fig. S is a an exploded perspective view of the system of the top of the cartridge bottom as seen in US 2006/0008847 A1 / WO 2005/108991 A2 (U.S. Patent Application 11/121,972).

Fig. 6 is an exploded perspective view of the present invention of the top of the cartridge bottom , showing novel changes made to the design of Fig. 5.

Fig. 7 is perspective illustration of the present invention.

### DETAILED DESCRIPTION OF THE DRAWINGS:

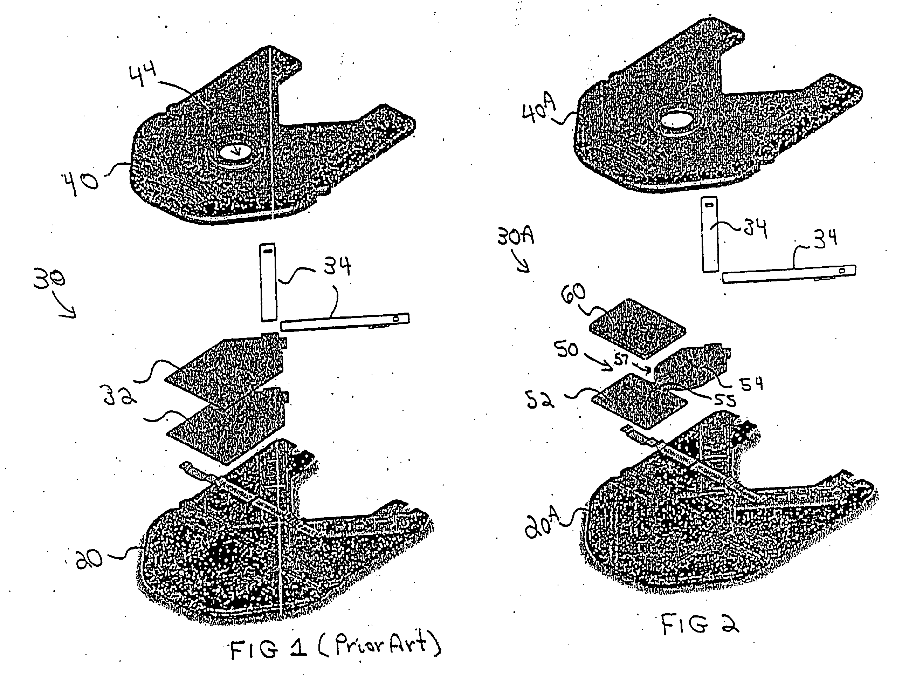

Embodiments of the system as set forth in US 2006/0008847 A1 / WO 2005/108991 A2 (U.S. Patent Application 11/121,972) are seen in Figs. 1, 3 and 5. The patentable novel improvements included in the present invention are seen in Figs. 2, 4, 6 and 7.

Fig. 1 shows a system 30 having a cartridge top 40 and bottom 20. One or more sample receiving pads 32 are disposed under sample receiving hole 44 in cartridge top 40.

Fig. 2 illustrates the present invention in which a system 30A for controlling fluid flow movement between a sample receiving pad and a test strip is provided. This system includes a sample receiving pad 50 having first and second portions (52 and 54) separated by a narrowed portion 55. An absorbent pad 60 is in contact with first portion 52 of sample receiving pad 50. A test strip (or test strips) 34 is in contact with second portion 54 of sample receiving pad 50, as shown. In one embodiment, an end of test strip(s) 34 overlaps second portion 54 of sample receiving pad 50.

Preferably, absorbent pad 60 is in overlapping contact with first portion 52 of sample receiving pad 50. For example, absorbent pad 60 may be positioned on top of first portion 52 of sample receiving pad 50. Sample receiving pad 50, absorbent pad 60 and test strip(s) 34 may all be disposed in a cartridge, as shown.

In an optional aspect, second portion 54 of sample receiving pad 50 may have an inwardly curved rear edge 57 adjacent to narrowed portion 55 of sample receiving pad 50 that separates first and second portions 52 and 54.

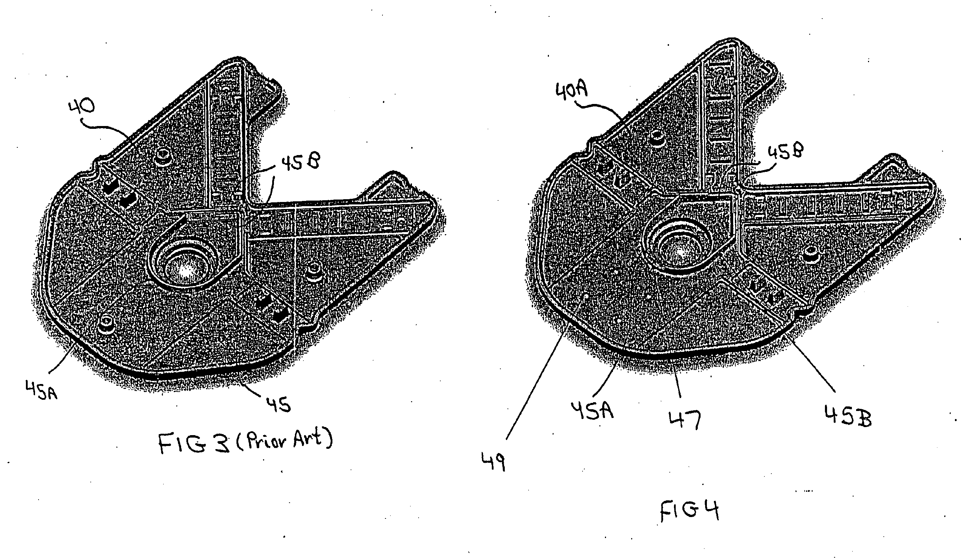

Fig. 3 shows the system of the underside of the cartridge top 40 as seen in US 2006/0008847 A1 / WO 2005/108991 A2 (U.S.Patent Application 11/121,972). A pinch wall 45 (comprising first portion 45A and second portion 45B) is provided.

Fig. 4 illustrates the present invention in which cartridge top 40A also has a pinch wall 45 (comprising first portion 45A and second portion 45B). Similar to the embodiment shown in Fig. 3, pinch wall 45 compresses sample receiving pad 50. In addition, pinch wall 45 pay comprise: a first portion 45A separating a sample receiving portion of the sample receiving pad 50 from a portion of the sample receiving pad 50 positioned away from test strip(s) 34, and a second portion 45B separating the sample receiving portion of the sample receiving pad 50 from a portion of the sample receiving pad 50 positioned adjacent to test strip(s) 34, wherein second portion 45B of the pinch wall compresses the sample receiving pad to a lesser degree than the first portion 45A of sample receiving pad 50.

As such, pinch wall 45 compresses the sample receiving pad 50 such that fluid received onto the sample receiving pad 50 preferentially tends to flow from the sample receiving portion of the sample receiving pad into the portion of the sample receiving pad positioned adjacent to test strip(s) 34, and less so from the sample receiving portion of the sample receiving pad 50 to the portion of the sample receiving pad positioned away from test strip(s) 34.

In optional aspects, first portion 45A of the pinch wall compresses 60 to 90% of the height of the sample receiving pad 50 and second portion 45B of the pinch wall compresses 2 to 30% of the height of the sample receiving pad. In other optional aspects, the first portion 45A of the pinch wall compresses 70 to 80% of the height of the sample receiving pad 50 and the second portion 45B of the pinch wall compresses 5 to 15% of the height of the sample receiving pad 50.

In accordance with the present invention, pinch wall portion 45A includes a notch 47. Notch 47 permits fluid to flow directly into absorbent pad 60. Thus, any excess fluid sample sitting on top of second portion 54 can be absorbed directly into absorbent pad 60. This feature of the invention helps to ensure that test strips 34 do not become flooded. Thus, excess sample volumes can be adequately handled (i.e.: by increasing the overflow capacity of the device).

As can also be seen in Fig. 4, optional wick pad compression dimples 49 can be used to hold absorbent pad 60 in place.

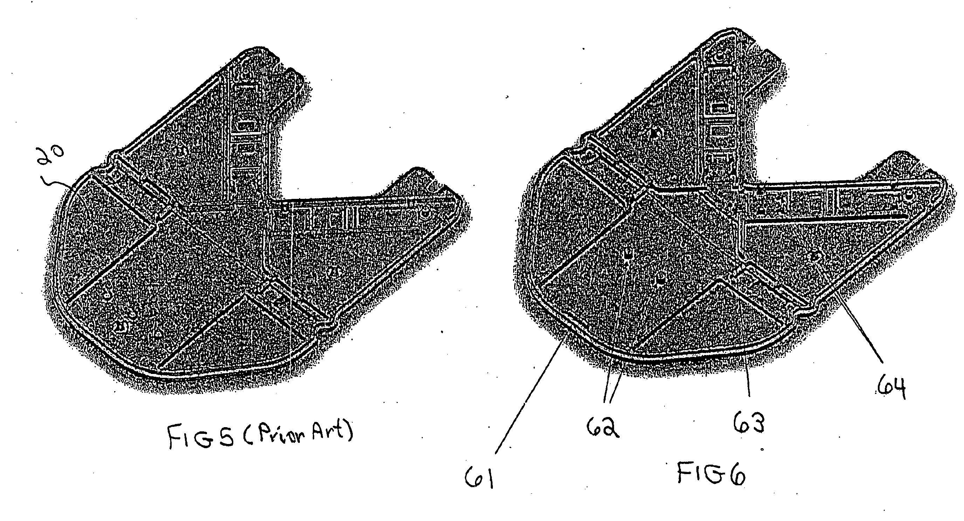

Fig. 5 shows the system of the top of the cartridge bottom 20 as seen in US 2006/0008847 A1 / WO 2005/108991 A2 (U.S. Patent Application 11/121,972). Fig. 6 illustrates optional features of the present invention including a wick pad positioning wall 6 1 and sample pad positioning pins 62 that can be used to hold absorbent pad 60 in place. Also included are a raised sample pad floor 63 which advantageously permits smaller amounts of fluid to be used in the present invention (without flooding test strip(s) 34). Also included are strip ramps 64 for positioning test strip(s) 34.

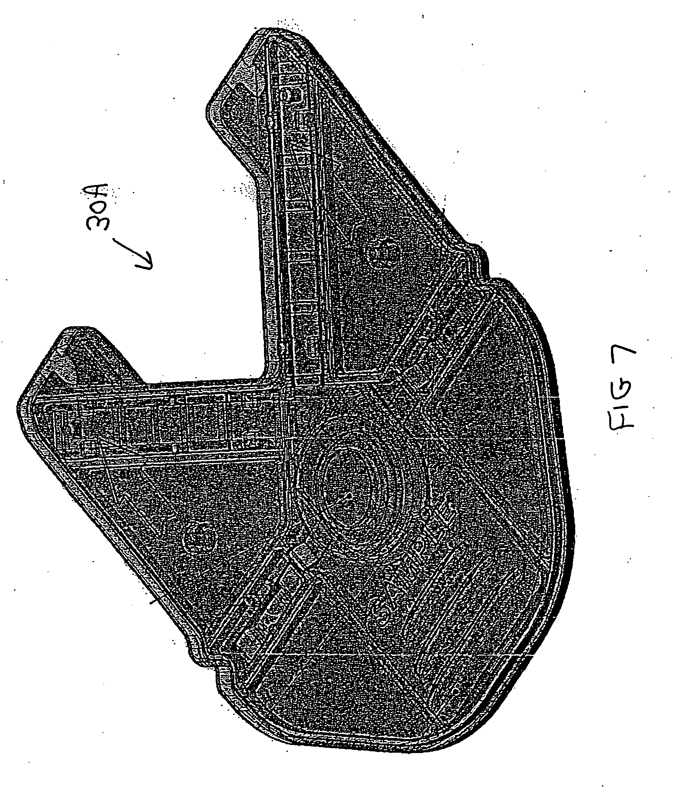

Lastly, Fig. 7 illustrates a view through assembled system 30A.

## Claims

1. A system (30) for controlling fluid flow movement between a sample receiving pad (50) and a test strip (34), comprising:
a sample receiving pad (50) having first and second portions (52, 54) separated by a narrowed portion (55);
an absorbent pad (60) in contact with the first portion (52) of the sample receiving pad (50); and
a test strip (34) in contact with the second portion (54) of the sample receiving pad (50).

2. The system (30) of claim 1, wherein the absorbent pad (60) is in overlapping contact with the first portion (52) of the sample receiving pad (50).

3. The system (30) of claim 2, wherein the absorbent pad (60) is positioned on top of the first portion (52) of the sample receiving pad (50).

4. The system (30) of one of the claims 1 to 3, wherein the sample receiving pad (50), absorbent pad (60) and test strip (34) are all disposed in a cartridge.

5. The system (30) of claim 4, wherein the cartridge comprises a body having a sample receiving hole (44) positioned over the second portion (54) of the sample receiving pad (50).

6. The system (30) of one of the claims 1 to 5, wherein an end of the test strip (34) overlaps the second portion (54) of the sample receiving pad (50).

7. The system (30) of one of the claims 1 to 6, wherein the second portion (54) of the sample receiving pad (50) has an inwardly curved rear edge (57) adjacent to the narrowed portion (55) of the sample receiving pad (50) separating the first and second portions (52, 54).

8. The system (30) of one of the claims 1 to 7, further comprising: a second test strip (34) in contact with the second portion (54) of the sample receiving pad (50).

9. The system (30) of claim 4, wherein the cartridge comprises a pinch wall (45) compressing the sample receiving pad (50).

10. The system (30) of claim 9, wherein the pinch wall (45) comprises: a first portion (45A) separating a sample receiving portion of the sample receiving pad (50) from a portion of the sample receiving pad (50) positioned away from the test strip (34), and a second portion (45B) separating the sample receiving portion of the sample receiving pad (50) from a portion of the sample receiving pad (50) positioned adjacent to the test strip (34), wherein the second portion (45B) of the pinch wall (45) compresses the sample receiving pad (50) to a lesser degree than the first portion (45A).

11. The system (30) of claim 10, wherein the first and second portions (45A,B) of the pinch wall (45) together continuously surround the sample receiving portion of the sample receiving pad (50).

12. The system (30) of claim 10 or 11, wherein the pinch wall (45) compresses the sample receiving pad (50) such that fluid received onto the sample receiving pad (50) preferentially tends to flow from the sample receiving portion of the sample receiving pad (50) into the portion of the sample receiving pad positioned adjacent to the test strip (34), and less so from the sample receiving portion of the sample receiving pad (50) to the portion of the sample receiving pad (50) positioned away from the test strip (34).

13. The system (30) of one of the claims 10 to 12, wherein the first portion (45A) of the pinch wall (45) compresses 60 to 90% of the height of the sample receiving pad (50) and the second portion of the pinch wall (45) compresses 2 to 30% of the height of the sample receiving pad (50).

14. The system (30) of one of the claims 10 to 13, wherein the first portion (45A) of the pinch wall (45) compresses 70 to 80% of the height of the sample receiving pad (50) and the second portion (45B) of the pinch wall (45) compresses 5 to 15% of the height of the sample receiving pad (50).

15. The system (30) of one of the claims 10 to 14, wherein the first portion (45A) of the pinch wall (30) comprises a notch (47) adjacent to the absorbent pad (60) to permit fluid flow into the absorbent pad (60).

## Patentansprüche

1. System (30) zum Steuern einer Fluidströmungsbewegung zwischen einer Probenaufnahmeplatte (50) und einem Teststreifen (34), mit:
einer Probenaufnahmeplatte (50), die einen ersten und einen zweiten Abschnitt (52, 54) besitzt, die durch einen schmäleren Abschnitt (55) getrennt sind;
einer Absorptionsmittelplatte (60), die mit dem ersten Abschnitt (52) der Probenaufnahmeplatte (50) in Kontakt ist; und
einem Teststreifen (34), der mit dem zweiten Abschnitt (54) der Probenaufnahmeplatte (50) in Kontakt ist.

2. System (30) nach Anspruch 1, wobei die Absorptionsmittelplatte (60) in einem überlappenden Kontakt mit dem ersten Abschnitt (52) der Probenaufnahmeplatte (50) ist.

3. System (30) nach Anspruch 2, wobei die Absorptionsmittelplatte (60) auf dem ersten Abschnitt (52) der Probenaufnahmeplatte (50) positioniert ist.

4. System (30) nach einem der Ansprüche 1 bis 3, wobei die Probenaufnahmeplatte (50), die Absorptionsmittelplatte (60) und der Teststreifen (34) sämtlich in einer Kassette angeordnet sind.

5. System (30) nach Anspruch 4, wobei die Kassette einen Körper mit einem Probenaufnahmeloch (44), das über dem zweiten Abschnitt (54) der Probenaufnahmeplatte (50) positioniert ist, enthält.

6. System (30) nach einem der Ansprüche 1 bis 5, wobei ein Ende des Teststreifens (34) mit dem zweiten Abschnitt (54) der Probenaufnahmeplatte (50) überlappt.

7. System (30) nach einem der Ansprüche 1 bis 6, wobei der zweite Abschnitt (54) der Probenaufnahmeplatte (50) eine nach innen gekrümmte Hinterkante (57) benachbart zu dem schmäleren Abschnitt (55) der Probenaufnahmeplatte (50), der den ersten von dem zweiten Abschnitt (52, 54) trennt, besitzt.

8. System (30) nach einem der Ansprüche 1 bis 7, ferner mit: einem zweiten Teststreifen (34), der mit dem zweiten Abschnitt (54) der Probenaufnahmeplatte (50) in Kontakt ist.

9. System (30) nach Anspruch 4, wobei die Kassette eine Klemmwand (45) aufweist, die die Probenaufnahmeplatte (50) zusammendrückt.

10. System (30) nach Anspruch 9, wobei die Klemmwand (45) Folgendes enthält: einen ersten Abschnitt (45A), der einen Probenaufnahmeabschnitt der Probenaufnahmeplatte (50) von einem Abschnitt der Probenaufnahmeplatte (50), der entfernt von dem Teststreifen (34) positioniert ist, trennt, und einen zweiten Abschnitt (45B), der den Probenaufnahmeabschnitt der Probenaufnahmeplatte (50) von einem Abschnitt der Probenaufnahmeplatte (50), der benachbart zu dem Teststreifen (34) positioniert ist, trennt, wobei der zweite Abschnitt (45B) der Klemmwand (45) die Probenaufnahmeplatte (50) in einem geringeren Ausmaß als den ersten Abschnitt (45A) zusammendrückt.

11. System (30) nach Anspruch 10, wobei der erste und der zweite Abschnitt (45A,B) der Klemmwand (45) gemeinsam den Probenaufnahmeabschnitt der Probenaufnahmeplatte (50) ununterbrochen umgeben.

12. System (30) nach Anspruch 10 oder 11, wobei die Klemmwand (45) die Probenaufnahmeplatte (50) zusammendrückt, so dass Fluid, das auf der Probenaufnahmeplatte (50) aufgenommen ist, vorzugsweise bestrebt ist, von dem Probenaufnahmeabschnitt der Probenaufnahmeplatte (50) in den Abschnitt der Probenaufnahmeplatte, der benachbart zu dem Teststreifen (34) positioniert ist, zu strömen, und daher weniger von dem Probenaufnahmeabschnitt der Probenaufnahmeplatte (50) zu dem Abschnitt der Probenaufnahmeplatte (50), der entfernt von dem Teststreifen (34) positioniert ist, strömt.

13. System (30) nach einem der Ansprüche 10 bis 12, wobei der erste Abschnitt (45A) der Klemmwand (45) 60 bis 90 % der Höhe der Probenaufnahmeplatte (50) zusammendrückt und der zweite Abschnitt der Klemmwand (45) 2 bis 30 % der Höhe der Probenaufnahmeplatte (50) zusammendrückt.

14. System (30) nach einem der Ansprüche 10 bis 13, wobei der erste Abschnitt (45A) der Klemmwand (45) 70 bis 80 % der Höhe der Probenaufnahmeplatte (50) zusammendrückt und der zweite Abschnitt (45B) der Klemmwand (45) 5 bis 15 % der Höhe der Probenaufnahmeplatte (50) zusammendrückt.

15. System (30) nach einem der Ansprüche 10 bis 14, wobei der erste Abschnitt (45A) der Klemmwand (30) eine Kerbe (47) benachbart zu der Absorptionsmittelplatte (60) aufweist, um eine Fluidströmung in die Absorptionsmittelplatte (60) zuzulassen.

## Revendications

1. Dispositif (30) qui contrôle le déplacement d'écoulement d'un fluide entre un tampon (50) de réception d'un échantillon et une bandelette réactive (34), comprenant :
un tampon (50) de réception d'un échantillon qui présente une première et une deuxième partie (52, 54) séparées par une partie rétrécie (55),
un tampon absorbant (60) en contact avec la première partie (52) du tampon (50) de réception d'un échantillon et
une bandelette réactive (34) en contact avec la deuxième partie (54) du tampon (50) de réception d'un échantillon.

2. Dispositif (30) selon la revendication 1, dans lequel le tampon absorbant (60) est en contact de superposition avec la première partie (52) du tampon (50) de réception d'un échantillon.

3. Dispositif (30) selon la revendication 2, dans lequel le tampon absorbant (60) est positionné au sommet de la première partie (52) du tampon (50) de réception d'un échantillon.

4. Dispositif (30) selon l'une quelconque des revendications 1 à 3, dans lequel le tampon (50) de réception d'un échantillon, le tampon absorbant (60) et la bandelette réactive (34) sont tous placés dans une cartouche.

5. Dispositif (30) selon la revendication 4, dans lequel la cartouche comprend un corps qui présente un orifice (44) de réception d'un échantillon placé au-dessus de la deuxième partie (54) du tampon (50) de réception d'un échantillon.

6. Dispositif (30) selon l'une quelconque des revendications 1 à 5, dans lequel une extrémité de la bandelette réactive (34) est superposée à la deuxième partie (54) du tampon (50) de réception d'un échantillon.

7. Dispositif (30) selon l'une quelconque des revendications 1 à 6, dans lequel la deuxième partie (54) du tampon (50) de réception d'un échantillon présente une arête arrière (57) courbée vers l'intérieur à côté de la partie rétrécie (55) du tampon (50) de réception d'un échantillon, cette arête séparant la première et la deuxième partie (52, 54).

8. Dispositif (30) selon l'une quelconque des revendications 1 à 7, comprenant de plus une deuxième bandelette réactive (34) en contact avec la deuxième partie (54) du tampon (50) de réception d'un échantillon.

9. Dispositif (30) selon la revendication 4, dans lequel la cartouche comprend une paroi de serrage (45) qui comprime le tampon (50) de réception d'un échantillon.

10. Dispositif (30) selon la revendication 9, dans lequel la paroi (45) de serrage comprend : une première partie (45A) qui sépare une partie de réception d'un échantillon du tampon (50) de réception d'un échantillon d'une partie du tampon (50) de réception d'un échantillon située à distance de la bandelette réactive (34) et une deuxième partie (45B) qui sépare la partie de réception d'un échantillon du tampon (50) de réception d'un échantillon d'une partie du tampon (50) de réception d'un échantillon située à côté de la bandelette réactive (34), la deuxième partie (45B) de la paroi de serrage (45) comprimant le tampon (50) de réception d'un échantillon à un degré moindre que la première partie (45A).

11. Dispositif (30) selon la revendication 10, dans lequel la première et la deuxième partie (45A, 45B) de la paroi de serrage (45) entourent ensemble de façon continue la partie de réception d'un échantillon du tampon (50) de réception d'un échantillon.

12. Dispositif (30) selon la revendication 10 ou 11, dans lequel la paroi de serrage (45) comprime le tampon (50) de réception d'un échantillon de telle sorte que le fluide reçu dans le tampon (50) de réception d'un échantillon tende à s'écouler préférentiellement de la partie de réception d'un échantillon du tampon (50) de réception d'un échantillon à la partie du tampon de réception d'un échantillon située à côté de la bandelette réactive (34) et que le fluide ait moins tendance à s'écouler de la partie de réception d'un échantillon du tampon (50) de réception d'un échantillon à la partie du tampon (50) de réception d'un échantillon située à distance de la bandelette réactive (34).

13. Dispositif (30) selon l'une quelconque des revendications 10 à 12, dans lequel la première partie (45A) de la paroi de serrage (45) comprime de 60 à 90 % la hauteur du tampon (50) de réception d'un échantillon et la deuxième partie de la paroi de serrage (45) comprime de 2 à 30 % la hauteur du tampon (50) de réception d'un échantillon.

14. Dispositif (30) selon l'une quelconque des revendications 10 à 13, dans lequel la première partie (45A) de la paroi de serrage (45) comprime de 70 à 80 % de la hauteur du tampon (50) de réception d'un échantillon et la deuxième partie (45B) de la paroi de serrage (45) comprime de 5 à 15 % de la hauteur du tampon (50) de réception d'un échantillon.

15. Dispositif (30) selon l'une quelconque des revendications 10 à 14, dans lequel la première partie (45A) de la paroi de serrage (30) comprend une entaille (47) située à côté du tampon absorbant (60) afin de permettre l'écoulement de fluide vers le tampon absorbant (60).
